# EUROPEAN PATENT SPECIFICATION

(11) **EP 2 651 282 B1**
(45) Date of publication and mention of the grant of the patent: **05.04.2017**
(21) Application number: 11807765.0
(22) Date of filing: 13.12.2011
(51) Int. Cl.: A61B 1/04, A61B 1/12

(54) **MAGNETICALLY GUIDED ROBOTIC DEVICE FOR ENDOSCOPIC PROCEDURES**
MAGNETISCH GEFÜHRTE ROBOTISCHE VORRICHTUNG FÜR ENDOSKOPISCHE VERFAHREN
DISPOSITIF ROBOTIQUE GUIDÉ DE FAÇON MAGNÉTIQUE POUR DES PROCÉDURES ENDOSCOPIQUES

(30) Priority: 13.12.2010 IT FI20100241
(43) Date of publication of application: 23.10.2013
(73) Proprietor: Scuola Superiore di Studi Universitari e di Perfezionamento Sant'Anna, 56125 Pisa (IT)
(72) Inventor: VALDASTRI, Pietro, I-57100 Livorno (IT); CIUTI, Gastone, I-56121 Pisa (IT); MENCIASSI, Arianna, I-56020 Pontedera (Pisa) (IT); DARIO, Paolo, I-57100 Livorno (IT)
(74) Representative: Brazzini, Silvia
(86) International application number: PCT/IB2011/055636
(87) International publication number: WO 2012/080947

(56) References cited:
- EP-A1- 1 698 268
- EP-A1- 1 992 271
- EP-A2- 1 310 207
- WO-A2-2007/086073
- DE-A1-102005 032 371
- DE-A1-102005 032 578

## Description

### Field of the invention

The present invention refers in general to the field of endoscopic devices. More precisely the invention concerns a magnetically guided robotic device of the capsular type for carrying out endoscopic procedures, in particular in the gastrointestinal tract.

### State of the art

Endoscopic procedures of the gastrointestinal tract use flexible probes with optical fibres equipped with powerful lens systems, a light source and operation channels for diagnosing the digestive lumen and possibly for the passage of drugs or instruments that are suitable for carrying out local therapeutic and/or surgical treatments. Although this medical procedure is the only procedure that is minimally invasive available to this day for diagnosing and treating pathologies of the gastrointestinal tract with high efficiency and reliability, it is not generally tolerated well by patients since the insertion of the flexible endoscopic probe, even though carried out through a natural orifice, and the blowing of air, generate pain. Pain is the most frequent complication that occurs while carrying out this diagnostic examination. In particular it can be caused by an excessive blowing of air, and also and especially by the formation of loops which pull at the mesentery thus causing pain. Indeed, if the operator is not capable of making the instrument move along the intestinal lumen correctly, it is possible for it to over-deform the intestine causing considerable pain to the patient. Moreover, conventional endoscopies can lead to complications due to sedation, cardio-pulmonary complications and impossibility of carrying out diagnostic examinations of certain portions of the digestive system, like the small intestine.

In order to solve such issues, by reducing the invasiveness and by enhancing the patient's compliance, capsular devices that are ingestible and can passively move in the gastrointestinal tract by effect of the peristaltic movements, represent the solution and the resource currently available on the market. Endoscopic capsules make it possible to obtain images of areas of interest of the gastrointestinal tract in a minimally invasive manner and without pain for the patient. The images thus obtained can be used by the doctor so as to detect lesions, polyps, areas of inner bleeding or for an early diagnosis of cancer of the gastrointestinal tract. The market leader in the field of endoscopic capsules is Given Imaging with the products PillCam System. Further important manufacturers of endoscopic capsules are Olympus (Endocapsule), IntroMedic Co. (MiroCam) and Chongqing Jinshan Science & Technology Group Co. Ltd (JS-MEII OMOM).

One of the main problems linked to the use of capsular endoscopic methods carried out through devices available on the market is the impossibility of spreading apart tissues while exploring tracts of the gastrointestinal system, such as colon, in which their reliability is greatly reduced because of the tendency of the tissues to collapse on the capsules. Generally, in such circumstances, the tissue completely wraps around the capsular system, limiting or preventing the lumen from being visualized. A further problem of the capsular endoscopy is related to the impossibility of exerting control on the direction and orientation of the camera mounted on the device. Commercial devices currently available on the market travel through the digestive tract passively through peristaltic contractions taking a few photos per second of the surrounding lumen with a direction and orientation that is completely random. This results in the possibility of having false negatives, since potential areas of interest may not be detected, and therefore in the impossibility of having an optimal and reliable diagnosis of the relevant digestive tract.

The need to overcome such problems, associated to the considerable advantage related to the reduction of invasiveness, has led to the development of a great amount of research aimed at improving the diagnostic abilities of the capsules by equipping them with robotic functions. In particular, the purpose is that of allowing an active motion and allowing them to intervene directly in the treatment of lesions. A very important aspect for the operation of a robotic capsule is its capability of spreading apart the tissues of the gastrointestinal tract travelled along, with the purpose of preventing them from being an obstacle to the movement of the capsule and of allowing a suitable visual exploration to be obtained.

An example of a capsular device that is capable of ensuring an active mechanical movement and a suitable widening of the lumen is described in WO2008/122997. In this document, for the spreading apart of the gastrointestinal tract tissues, the capsule was provided with extensible legs actuated by motors in direct current. If on one hand such a system allows both the movement of the capsule and the spreading apart of the surrounding tissue, on the other hand however, it requires a complex electromechanical device, which is costly and has high energy consumption. The capsule is equipped with two groups of six radially extensible legs which allow a uniform spreading apart of the collapsed tissue of the colon thus promoting the visualization of the inner surface and at the same time allowing the capsule to autonomously move. Even if the capsule according to the aforementioned patent has proven to be capable of travelling along the entire length of the large intestine, passing through narrow corners, such as the splenic flexure of the colon, it shows different technical problems as far as the supply system is concerned.

Another example of a capsular device with active movement is described in US2004181127. In this solution the control of the movements of the capsule is carried out by a magnetic system. On board of the capsule there are magnetic means or means responsive to an external magnetic field and the movement of the capsule in the intestinal lumen is induced through controlled movements of the external magnetic field. The movements of the capsule are however made difficult by the fact that it tends to stick to the wall of the lumen due to the magnetic force acting upon Document DE 10 2005 032 371 A1 discloses a capsular device according to the preamble of claim 1.

A complete review of the state of the art on the aspects of endoscopic capsules is given by Moglia A. et al, "Capsule endoscopy: progress update and challenges ahead", Nature Reviews Gastroenterology and Hepatology 6, 353-361 (June 2009).

The purpose of the present invention is to provide an endoscopic device of the capsular type which can avoid both the drawbacks of conventional flexible endoscopes, reducing the invasiveness thereof and the pain for the patient, thus increasing the patient's compliance, and the drawbacks of capsular endoscopes, by increasing their ability to control the movement and limiting their bulk.

A further purpose of the present invention is to provide an endoscopic device of the aforementioned type which also allows local therapeutic and/or surgical treatments to be carried out on areas of the inspected gastrointestinal tract.

### Summary of the invention

These and further purposes are achieved with the magnetically guided robotic device for endoscopic procedures according to the present invention whose main characteristics are reported in claim 1. Further important characteristics are reported in the dependent claims.

According to a main aspect of the invention, the present robotic device is of the capsular type equipped with an active control of its motion through a source, external to the patient, of magnetic field obtained with permanent magnetic systems or with electro magnets and it is in communication with an external unit for blowing air, which determines the spreading apart of the surrounding tissue, and for sucking fluids and substances present in the digestive tract. The blowing system can be also exploited for therapeutic purposes for releasing and dispensing a drug inside the gastrointestinal tract under examination and can be adapted for the introduction of medical instruments so as to carry out local surgery or for taking biopsy samples.

The possibility of developing a valid blowing system suitable for spreading apart the collapsed tissue of the gastrointestinal tract thus has a double clinical value for a robotic endoscopic system: (a) improvement of the visualization of the lumen by the on board camera, and (b) smoother passing of the capsule by using strategies of active movement.

The implementation of the wire-like endoscopic device according to the invention makes it possible to spread apart the collapsed tissue and consequently effectively visualize the lumen and allows active movement through the magnetic approach for diagnostic, therapeutic and surgical endoscopic procedures, of the minimally invasive type, representing a valid alternative to both conventional and capsular type endoscopy.

### Brief description of the drawings

Further characteristics and advantages of the robotic device for endoscopic procedures according to the present invention shall become clearer from the following description of an embodiment thereof given as an example and not for limiting purposes with reference to the attached drawings, in which:
figure 1 is a perspective view of an endoscopic capsule of the robotic device according to the invention;
figure 2 is a longitudinal section view of the capsule of figure 1;
figures from 3 to 7 schematically show the modalities of use of the robotic device according to the invention;
figure 8 is a perspective view of a first embodiment of the endoscopic capsule of the robotic device according to the invention;
figure 9 is a perspective view of a second embodiment of the endoscopic capsule of the robotic device according to the invention;
figure 10 is a longitudinal section view of the capsule of figure 9.

### Detailed description of the invention

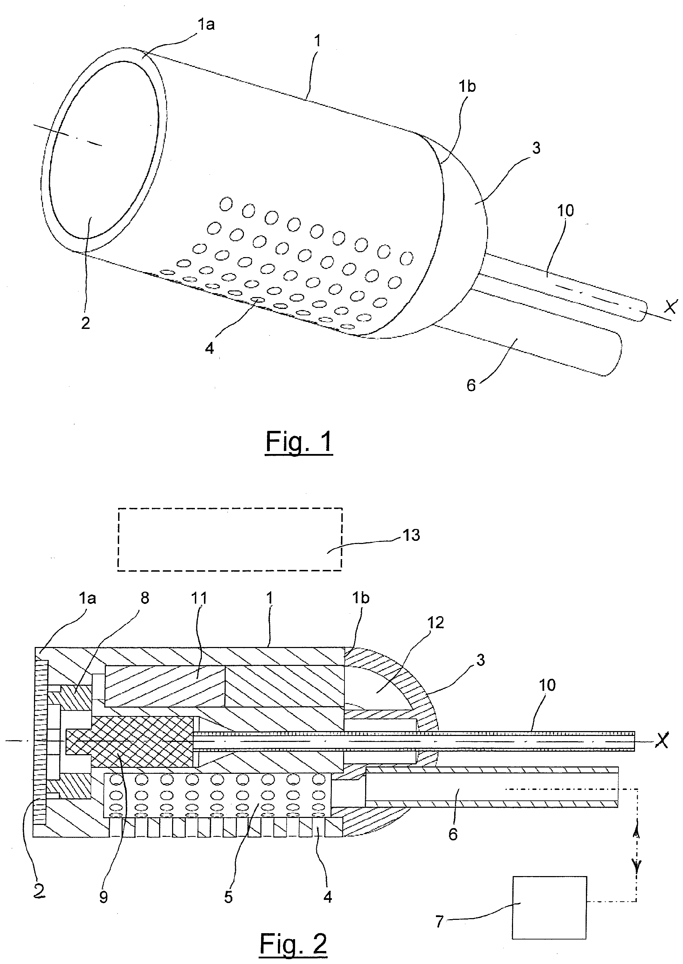

With reference to figure 1, the robotic device for endoscopic procedures according to the invention comprises a cylindrical hollow body 1, extending according to the longitudinal axis X, with dimensions that are comparable to those of an endoscopic capsule available on the market, i.e. about 11 mm in diameter and 24-31 mm (on average 26 mm) in length and therefore such as to allow it to be inserted, without difficulty, in the gastrointestinal tract through a natural orifice.

In the present embodiment of the invention the robotic device is configured so as to be used as a colonoscope, but it is clear that, with constructive modifications that are obvious to a person skilled in the art, it can be configured also for other endoscopic procedures, for example for gastroesophageal endoscopy.

The cylindrical body 1 is made from a biocompatible material, resistant to chemical agents present inside the large intestine, for example polyolefinic materials, such as high density polyethylene (HDPE), or fluoropolymer materials, such as polytetrafluoroethylene (PTFE). The thickness of the capsule body has to be such as to withstand an internal pressure of the order of kPa.

The cylindrical body 1 has a first end or front end 1 a and a second end or rear end 1b. The front end 1a is closed by a lens 2, flat in the illustrated embodiment, but it can also be configured as a cap or in any case that can be covered by a rounded end so as to facilitate the insertion and the movement inside the body cavity. The rear end 1b is closed by a cap portion 3. In the present description the terms front and rear must be intended as referring to the direction of movement of the capsular body 1 in the intestinal lumen.

As an example embodiment, the cylindrical body 1 has been made in acrylic material (VisiJet XT 200, Inition, ThingLab, London, UK) through rapid prototyping techniques. In particular such a material consists of one part (35-45%) of urethane acrylate polymer, a second part (45-55%) of triethylene glycol dimethacrylate ester, and it has a diameter of 15 mm, a length of 34 mm and a weight of 7.8 g. The capsule underwent sterilization through procedure with ethylene oxide without suffering any damages to the material or to the integrated electronics.

With reference also to figure 2, a plurality of holes 4 is formed on a part of the side surface of the body 1 so as to place the outside of the body 1 in communication with an inner chamber 5 thereof. The chamber 5 in turn communicates with a system for blowing a gaseous fluid, such as air, and for sucking, generically indicated with 7, in a use intended to remain external, of the conventional type, for example consisting of an air/water diaphragm pump distributed by the company Parker Hannifin, through a conduit or a flexible tube-shaped sheath 6 extending from the rear end 1 a of the body 1 through the cap 3. The air coming from the outer blowing system 7 is conveyed into the chamber 5 through the sheath 6 and is expelled in the intestinal lumen through the holes 4 for spreading apart the wall of the tract through which the capsule moves.

The flow of air is regulated through a control system, which is not shown, for example a flow-rate adjustment valve, and it is monitored in a closed cycle through flow and pressure sensors. Once the valve is open, the air can flow through the flexible sheath 6 to the chamber 5 where it is immediately expelled by means of the holes 4 inside the digestive tract. Alternatively, it is possible to create a depression and suck fluids or substances present in the intestinal tract. Moreover, the sheath 6 can be used to introduce drugs and consequently expel them onto the walls of the lumen for therapeutic purposes.

According to a preferred embodiment of the invention, a further flexible conduit is arranged parallel to the conduit 6 and extending through the cap 3 towards the front end 1 a; the further conduit is provided with a nozzle for spraying water on the lens 2 thus cleaning it from any materials possibly deposited on it during colonoscopic procedure. The water for this washing system comes from an outer system conveying water into the conduit through the nozzle, and it is expelled through the holes 4.

Near to the front end, in a position immediately behind the septum 2 there is an LED illumination group 8 and a camera 9 and relative optics for visualising the intestinal lumen in which there is the capsule. A second flexible sheath 10, for example around 1 mm in diameter, extends from the cap 3 and contains electric power supply wires for the LED, for the camera and for the control electronics present on the device, and an electrical wire for transmitting the video signal.

A magnetic element 11 is housed inside the body 1 in a further chamber 12 thereof extending longitudinally from the opposite side of the chamber 5 with respect to the axis X. The magnetic element 11 is a permanent magnet or an electromagnet with magnetization properties and direction that are compatible with external means generating an external magnetic field, generically indicated with reference numeral 13, so as to ensure an optimal alignment, dragging and orientation of the capsule in the digestive tract in question.

As an example, the magnetic element 11 inside the capsule can be constituted by a series of neodymium magnets (grade N52) with a circular shape and axial direction of magnetization and with a remanence equal to 1.48T. The single component of such an inner magnetic element has a diameter of 3 mm, a thickness of 1 mm and a weight equal to 0.05 g. Such magnetic modules are connected so as to make up three lines, each made up of 22 single magnetic components. The external magnetic field generator means 13 can, on the other hand, consist of a single neodymium magnet (grade N48 and remanence equal to 1.42T) having cylindrical shape with diametral magnetization direction and a diameter equal to 90 mm, a length equal to 100 mm and a weight of around 4 kg. The movement and the variation of orientation of such a magnet, as a function of the suitable selection of the properties of the magnetic modules, determine a corresponding variation of position and orientation of the capsular device inside the digestive tract.

The operation of the robotic device according to the invention is now described with reference to figures from 3 to 7.

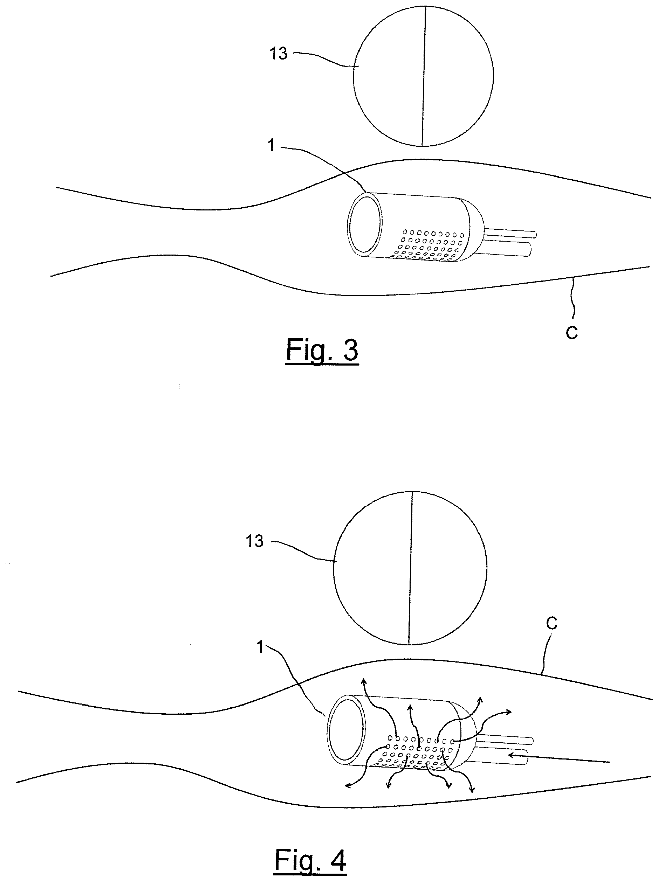

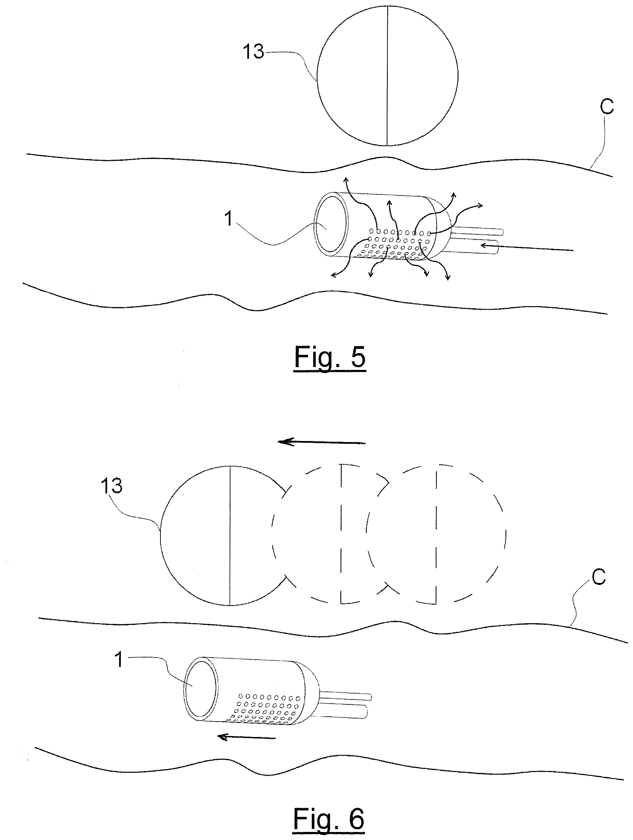

The capsule 1 is inserted in the body of the patient through a natural orifice (for example the anal channel in the case of a colonoscopy) and is dragged and oriented through magnetic coupling which is established between the external magnet 13 and the magnet 11 inside the capsule 1 in areas of the digestive tract C that are sufficiently spread apart so as to have a reliable motion and a good visualization of the lumen (figure 3). When the capsule comes up against collapsed regions, the blowing system is activated so as to stretch the surrounding tissue (figure 4). The compressed air, produced by the external unit 7, is introduced inside the flexible conduit 6, so as to be conveyed to the chamber 5 inside the capsule from where it is immediately expelled through the holes 4 present on the outer wall (figure 5).

Once the surrounding tissue has been stretched following the controlled blowing of air from the outside, the capsule can be transported and oriented in a reliable and effective manner through the magnetic coupling (figure 6) with the external magnet 13, whose movement produces a corresponding movement of the capsule.

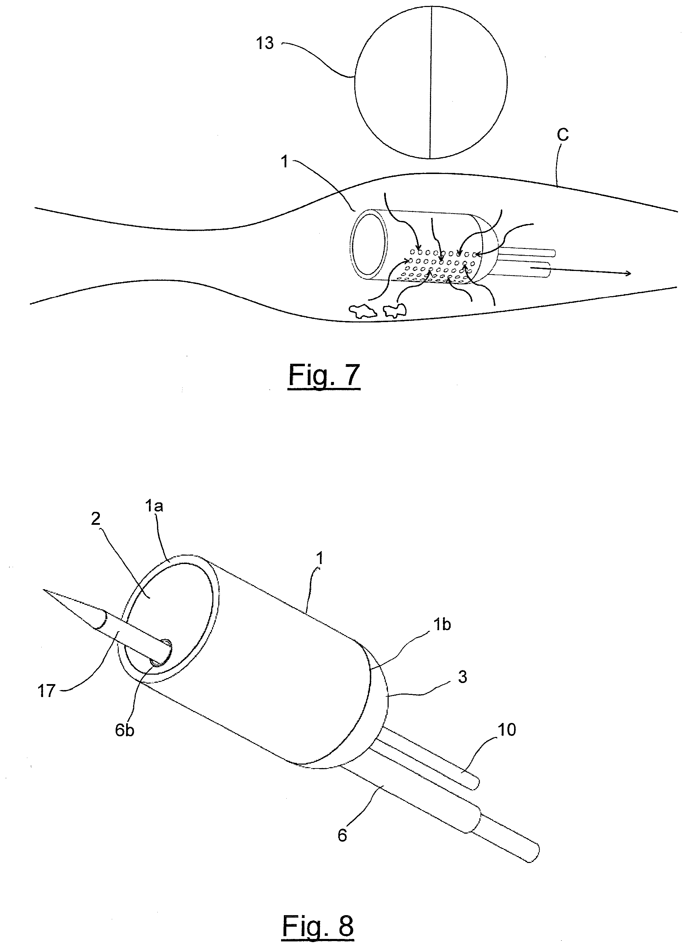

When necessary, the device is capable of sucking fluids and substances present in the digestive tract through the same path, in which the flexible conduit 6 is used to convey the sucked material outside (figure 7). The same blowing and sucking system may be used for therapeutic procedures through the localized dispersion of a drug inside the gastrointestinal tract.

The flexible conduit 6, used for the introduction of air and the consequent spreading apart of the tissue, as well as for the release of a drug and the suction of substances and fluids present in the digestive tract, can also be exploited as an operative channel for the passage of instruments such as pincers or devices for biopsy (in this case with an inner diameter that is not smaller than 2 mm for the passage of the endoscopic instruments).

This embodiment is illustrated in figure 8. The robotic device according to this embodiment can be used to carry out surgical procedures through a suitable instrument for endoscopic surgery, schematically illustrated and indicated with reference numeral 17. In this case the introduction and the suction of fluids, as well as the release of a drug in the gastrointestinal tract, does not occur through holes on the external wall, but rather the introduction and suction occur directly through the same flexible conduit 6 passing inside the capsule with an outlet 6b in the front end 1 a thereof, as shown in figure 8.

According to a particular embodiment of the invention, the conduit 6 is provided for having a variable flexibility and stiffness along its length, so that the part of the conduit coming out from the cap 3 comprises at least a first portion adjacent to the cap 3, that has higher bending flexibility, and a second portion comprised between the first one and the end of the conduit, where instruments are to be inserted, having a lower bending flexibility and a greater mechanical resistance than the rest of the conduit, the greater resistance and lower bending flexibility making easier the introduction of instruments. Moreover, this more rigid portion of the conduit is useful for possibly pulling or pushing the capsule by mechanical means, to be associated to the magnetic means in particular as a safety system for the rapid removal of the capsule from intestine. The first portion contiguous to the cap, thanks to its higher flexibility, facilitates the orientation of the capsular body 1, and may have approximately the same length as the capsular body, the second more rigid portion having different lengths according to different needs and uses of the capsule.

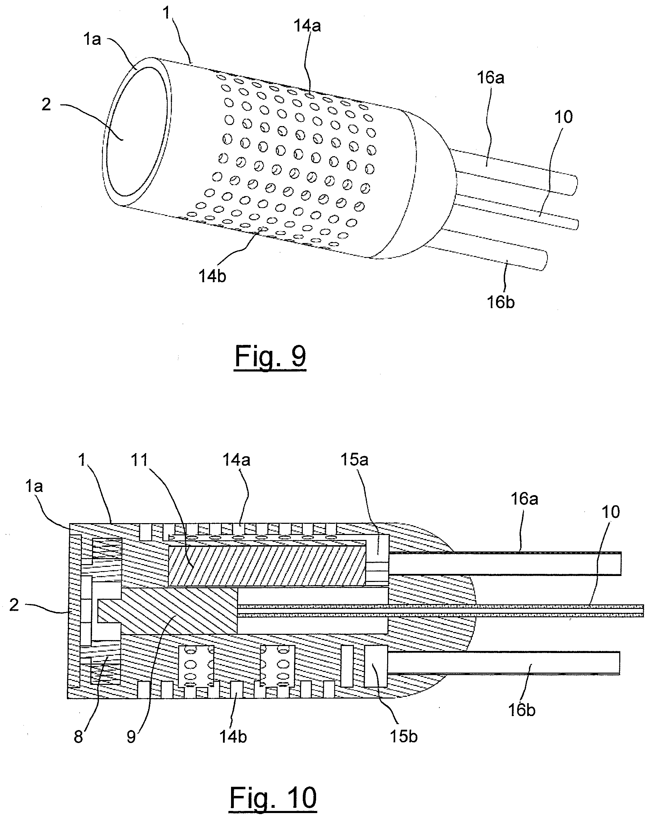

In another embodiment the blowing and sucking system is exploited so as to generate an air cushion between the capsule and the tissue so as to reduce the static and dynamic friction during the dragging and the orientation of the capsule. As shown in figures 9 and 10, the body 1 of the capsule comprises two inner chambers 15a and 15b, one containing the magnet 11, which receives air from outside through a first flexible conduit 16a and expels it into the lumen through holes 14a formed on the part of the capsular body in contact with the tissue, whereas the other one sucks the same amount of air blown, through a second flexible conduit 16b and holes 14b formed on the wall of the capsule free from the tissue, since it is opposite with respect to the part where the magnet 11 is arranged.

Such a process thus ensures the creation of an air cushion between the capsule 1 and the wall of the gastrointestinal tract in a condition of constant pressure inside the intestine. Once the tract under examination has been blown with a quantity of air sufficient for the spreading apart thereof (for ensuring the visualization and motion) the actuation of such a system considerably reduces the contact friction between the capsular device and the lumen itself. Indeed the blowing of air occurs in opposition with respect to the magnetic force, and therefore the capsular body 1 is prevented from totally sticking to the wall of the cavity, which could otherwise obstruct its movement. Also in this embodiment the same blowing system can be used for the localized release of a drug, whereas the suction system can be used for procedures of sucking fluids and substances inside the digestive tract.

The two flexible conduits 16a and 16b can be separated or joined inside a single channel connected to the external introduction or suction unit 7, and they are provided for having a variable flexibility and stiffness along their length as described above for the conduit 6.

The localization of the robotic device according to the invention inside the region of the large intestine can be controlled through magnetic tracking systems of the known type (like for example the systems described in Richert B. et al., "Magnetic sensor techniques for new intelligent endoscopic capsules" in 10th Symposium Magnetoresistive Sensors and Magnetic Systems, 2009; "Motilis medica." (http://www.motilis.com/)) to have an intuitive control of the capsule by the operator through the control platform. Finally, the presence of the flexible conduit 6 (or 16a, b) makes it possible to define the distance travelled by the capsule from the orifice in which it was inserted, and thus know the position of pathologies or clinically interesting spots for ensuring the possibility of a subsequent, possible operation.

The robotic device according to the present invention represents the first wire-like capsular robotic device for endoscopic procedures with active motion based upon magnetic fields and with a blowing system for spreading apart the digestive tissue and sucking fluids and substances present in the digestive tract, as well as for the localized release of drugs for therapeutic purposes.

The present device allows to reduce the invasiveness and the pain of endoscopic procedures carried out through conventional flexible endoscopes and the spreading apart of the tissue ensures an efficient and reliable visualization of the lumen and the possibility of motion through active magnetic approach. The device, in its embodiments mentioned above, can be used for diagnostic, therapeutic and surgical endoscopic procedures and represents a valid alternative to flexible endoscopes currently available on the market.

## Claims

1. Robotic device for endoscopic procedures comprising a capsular body (1) extending along a longitudinal axis (X), optoelectronic means (2, 9) for detecting images of a body cavity of a patient and means for lighting (8) said body cavity arranged in said capsular body (1), means responsive to a magnetic force integral to said capsular body (1) and comprising at least a magnet (11), means for generating a magnetic field (13) external to the patient, magnetically linkable to said means responsive to a magnetic force for moving and directing said capsular body (1) in said body cavity, and comprising at least a flexible conduit (6; 16a, 16b) for communicating said capsular body (1) with a unit (7) external to the patient for feeding a gaseous fluid to said cavity and sucking material therefrom, and means (4, 5; 6b; 14a,b, 15a,b) for blowing said gaseous fluid in said body cavity and sucking material or gaseous fluid present in said cavity, said means for blowing and sucking comprising at least one inner chamber (5, 15a) communicating with said flexible conduit and a plurality of through holes (4) formed on a portion of side wall of said capsular body (1) delimiting said chamber (5); **characterized in that** said at least one magnet (11) is arranged in a chamber (12) formed in the part of said capsular body radially opposed to said inner chamber (5, 15a) relative to said longitudinal axis (X) of said capsular body (1).

2. The robotic device according to any one of the previous claims, wherein said capsular body (1) comprises a first inner chamber (15a) delimited by a first portion of side wall of said capsular body (1) on which a first plurality of through holes (14a) is formed, and a second inner chamber (15b) housing said means responsive to a magnetic force (11) and delimited by a second portion of side wall of said capsular body (1), on which a second plurality of through holes (14b) is formed, a first (16a) and a second (16b) flexible conduit being provided for communicating said first (15a) and second (15b) inner chamber with said feeding/sucking unit (7), in such a way that said gaseous fluid is blown to said second inner chamber (15b) and said second conduit (16b), while material and gaseous fluid present in said cavity is sucked through said first inner chamber (15a) and said first conduit (16a), whereby an air cushion is formed between said capsular body (1) and the body cavity wall, in opposition to the magnetic force, thus reducing the friction.

3. The robotic device according to claim 2, wherein said flexible conduit (6) has such a size to allow the passage of a diagnostic or endoscopic surgery instrument (17) therethrough.

4. The robotic device according to any one of the previous claims, further comprising a wired electric connection (10) for electrically connecting said capsular body (1) to a power source external to the patient.

5. The robotic device according to any one of the previous claims, wherein said optoelectronic means comprise at least a lens (2) placed at one end of said capsular body (1) and a camera (9) for acquiring images of the cavity tract in which said capsular body (1) moves.

6. The robotic device according to any one of the previous claims, wherein said flexible conduit (6; 16a, 16b) is capable of conveying a drug to be locally dispensed in said body cavity.

7. The robotic device according to any one of the previous claims, wherein said body cavity is the gastrointestinal tract.

8. The robotic device according to any one of the previous claims, further comprising a further flexible conduit arranged parallel to the conduit (6; 16a, 16b) and extending through the cap (3) towards the front end (1 a), said further conduit being capable of supplying water on said lens (2) for washing it and cleaning from any materials possibly deposited on it during use.

9. The robotic device according to any one of the previous claims, wherein said flexible conduit (6, 16a, 16b) has a variable flexibility along its length so that the part of said conduit protruding from the cap (3) outside of the capsular body (1) comprises at least two portions having different flexibility, a first portion adjacent to said cap (3) having higher bending flexibility, and a second portion comprised between the first one and the end of said conduit having a lower bending flexibility and a higher mechanical resistance.

## Patentansprüche

1. Robotische Vorrichtung für endoskopische Verfahren, enthaltend einen kapselartigen Körper (1), der sich entlang einer Längsachse (X) erstreckt, optoelektronische Einrichtungen (2, 9) zum Detektieren von Bildern eines Körperhohlraumes eines Patienten und zum Beleuchten des Körperhohlraumes Einrichtungen (8), die in dem kapselartigen Körper (1) angeordnet sind, auf eine magnetische Kraft ansprechende Einrichtungen, die mit dem kapselartigen Körper (1) integral sind und wenigstens einen Magneten (11) enthalten, Einrichtungen (13) zum Erzeugen eines magnetischen Feldes außerhalb des Patienten, die magnetisch mit den auf eine magnetische Kraft ansprechende Einrichtungen verbindbar sind, um den kapselartigen Körper (1) in dem Körperhohlraum zu bewegen und zu dirigieren, und enthaltend wenigstens eine flexible Leitung (6; 16a, 16b), um den kapselartigen Körper (1) mit einer Einheit (7) außerhalb des Patienten in Verbindung zu setzen, um ein gasförmiges Fluid zu dem Hohlraum zuzuführen und Material daraus abzusaugen, und Einrichtungen (4, 5; 6b; 14a, b, 15a, b), um das gasförmige Fluid in den Körperhohlraum zu blasen und Material oder gasförmiges Fluid, das in dem Hohlraum vorhanden ist, abzusaugen, wobei die Einrichtungen zum Blasen und Absaugen wenigstens eine innere Kammer (5, 15a), die mit der flexiblen Leitung in Verbindung ist, und eine Mehrzahl von Durchgangslöchern (4) enthalten, die an einem Teil einer Seitenwand des kapselartigen Körpers (1) ausgebildet sind, die die Kammer (5) begrenzt;
**dadurch gekennzeichnet, dass** der wenigstens eine Magnet (11) in einer Kammer (12) angeordnet ist, die in dem Teil des kapselartigen Körpers ausgebildet ist, der der inneren Kammer (5, 15a) relativ zu der Längsachse (X) des kapselartigen Körpers (1) radial gegenüber liegt.

2. Robotische Vorrichtung nach einem der vorhergehenden Ansprüche, wobei der kapselartige Körper (1) eine erste innere Kammer (15a), die durch einen ersten Teil der Seitenwand des kapselartigen Körpers (1) begrenzt ist, an der eine erste Mehrzahl von Durchgangslöchern (14a) ausgebildet ist, und eine zweite innere Kammer (15b) enthält, die die Einrichtungen (11), die auf eine magnetische Kraft ansprechen, beherbergt und durch einen zweiten Teil der Seitenwand des kapselartigen Körpers (1) begrenzt ist, an der eine zweite Mehrzahl von Durchgangslöchern (14b) ausgebildet ist, wobei eine erste (16a) und eine zweite flexible Leitung (16b) vorgesehen sind, um die erste (15a) und zweite innere Kammer (15b) mit der Zuführ-/Absaug-Einheit (7) auf eine solche Weise in Verbindung zu setzen, dass das gasförmige Fluid zu der zweiten inneren Kammer (15b) und der zweigen Leitung (16b) geblasen wird, während Material und gasförmiges Fluid, die in dem Hohlraum vorhanden sind, durch die erste innere Kammer (15a) und die erste Leitung (16a) abgesaugt werden, wodurch zwischen dem kapselartigen Körper (1) und der Körperhohlraumwand entgegengesetzt zur magnetischen Kraft ein Luftpolster gebildet wird, womit die Reibung verringert wird.

3. Robotische Vorrichtung nach Anspruch 2, wobei die flexible Leitung (6) eine solche Größe hat, um den Durchgang eines diagnostischen oder endoskopischen Operationsinstruments (17) dadurch hindurch zu gestatten.

4. Robotische Vorrichtung nach einem der vorhergehenden Ansprüche, wobei ferner eine verkabelte elektrische Verbindung (10) enthalten ist, um den kapselartigen Körper (1) elektrisch mit einer Energiequelle außerhalb des Patienten zu verbinden.

5. Robotische Vorrichtung nach einem der vorhergehenden Ansprüche, wobei die optoelektronsichen Einrichtungen wenigstens eine Linse (2), die an einem Ende des kapselartigen Körpers (1) platziert ist, und eine Kamera (9) enthalten, um Bilder von dem Hohlraumtrakt zu erlangten, in dem sich der kapselartige Körper (1) bewegt.

6. Robotische Vorrichtung nach einem der vorhergehenden Ansprüche, wobei die flexible Leitung (6; 16a, 16b) geeignet ist, ein Medikament zuzuführen, das lokal in dem Körperhohlraum zu verabreichen ist.

7. Robotische Vorrichtung nach einem der vorhergehenden Ansprüche, wobei der Körperhohlraum ein gastrointestinaler Trakt ist.

8. Robotische Vorrichtung nach einem der vorhergehenden Ansprüche, wobei ferner eine weitere flexible Leitung enthalten ist, die parallel zu der Leitung (6; 16a, 16b) angeordnet ist und sich durch die Kappe (3) zum vorderen Ende (1a) hin erstreckt, wobei die weitere Leitung geeignet ist, Wasser auf die Linse (2) zuzuführen, um sie zu waschen und von jeglichen Materialien zu reinigen, die sich während der Verwendung möglicherweise auf ihr abgelagert haben.

9. Robotische Vorrichtung nach einem der vorhergehenden Ansprüche, wobei die flexible Leitung (6; 16a, 16b) über ihre Länge eine variable Flexibilität hat, so dass der Teil der Leitung, der von der Kappe (3) außerhalb des kapselartigen Körpers (1) vorsteht, wenigstens zwei Teile enthält, die unterschiedliche Flexibilitäten haben, wobei ein erster Teil benachbart der Kappe (3) eine höhere Biegeflexibilität hat und ein zweiter Teil, der zwischen dem ersten und dem Ende der Leitung enthalten ist, eine geringere Biegeflexibilität und einen höheren mechanischen Widerstand hat.

## Revendications

1. Dispositif robotisé pour des procédures endoscopiques, comprenant un corps capsulaire (1) s'étendant le long d'un axe longitudinal (X), des moyens optoélectroniques (2, 9) pour détecter des images d'une cavité corporelle d'un patient et des moyens d'éclairage (8) de ladite cavité corporelle agencés dans ledit corps capsulaire (1), des moyens sensibles à une force magnétique solidaires dudit corps capsulaire (1) et comprenant au moins un aimant (11), des moyens pour générer un champ magnétique (13) extérieur au patient, pouvant être liés magnétiquement auxdits moyens sensibles à une force magnétique (11) pour déplacer et diriger ledit corps capsulaire (1) dans ladite cavité corporelle, et comprenant au moins un conduit flexible (6; 16a, 16b) pour faire communiquer ledit corps capsulaire (1) avec une unité (7) extérieure au patient pour alimenter en fluide gazeux ladite cavité et aspirer de la matière dans celle-ci, et des moyens (4, 5; 6b; 14a,b, 15a,b) pour souffler ledit fluide gazeux dans ladite cavité corporelle et aspirer de la matière ou du fluide gazeux présent dans ladite cavité, lesdits moyens pour souffler et aspirer comprenant au moins une chambre intérieure (5, 15a) communiquant avec ledit conduit flexible et une pluralité d'orifices traversants (4) formés dans une partie de la paroi dudit corps capsulaire (1) délimitant ladite chambre (5) ; **caractérisé en ce que** ledit au moins un aimant (11) est disposé dans une chambre (12) formée dans la partie dudit corps capsulaire radialement opposée à ladite chambre intérieure (5, 15a) par rapport audit axe longitudinal (X) dudit corps capsulaire (1).

2. Le dispositif robotisé selon la revendication 1, dans lequel ledit corps capsulaire (1) comprend une première chambre intérieure (15a) délimitée par une première partie de paroi latérale dudit corps capsulaire (1) sur laquelle est réalisée une première pluralité d'orifices traversants (14a), et une seconde chambre intérieure (15b) logeant lesdits moyens sensibles à une force magnétique (11) et délimitée par une seconde partie de paroi latérale dudit corps capsulaire (1), sur laquelle est réalisée une seconde pluralité d'orifices traversants (14b), un premier (16a) et un second (16b) conduits flexibles étant prévus pour faire communiquer lesdites première (15a) et seconde (15b) chambres intérieures avec ladite unité d'alimentation/aspiration (7), de façon que ledit fluide gazeux est soufflé dans ladite seconde chambre intérieure (15b) et ledit second conduit (16b), tandis que la matière et le fluide présents dans ladite cavité sont aspirés via ladite première chambre intérieure (15a) et ledit premier conduit (16a), dans lequel est réalisé un coussin d'air entre ledit corps capsulaire (1) et la paroi de la cavité corporelle, en opposition à la force magnétique, réduisant ainsi les frottements.

3. Le dispositif robotisé selon la revendication 2, dans lequel ledit conduit flexible (6) présente une dimension pour permettre le passage, dans celui-ci, d'un instrument de chirurgie endoscopique ou de diagnostic (17).

4. Le dispositif robotisé selon l'une quelconque des revendications précédentes, comprenant en outre une connexion électrique câblée (10) pour connecter électriquement ledit corps capsulaire (1) à une source d'énergie extérieure au patient.

5. Le dispositif robotisé selon l'une quelconque des revendications précédentes, dans lequel lesdits moyens optoélectroniques comprennent au moins une lentille (2) placée à une extrémité dudit corps capsulaire (1) et une caméra (9) pour l'acquisition d'images dans le tractus cavité dans lequel se déplace ledit corps capsulaire (1).

6. Le dispositif robotisé selon l'une quelconque des revendications précédentes, dans lequel ledit conduit flexible (6; 16a, 16b) est apte à transporter un médicament devant être délivré localement dans ladite cavité corporelle.

7. Le dispositif robotisé selon l'une quelconque des revendications précédentes, dans lequel ladite cavité corporelle est le tractus gastro-intestinal.

8. Le dispositif robotisé selon l'une quelconque des revendications précédentes, comprenant en outre un conduit flexible supplémentaire disposé parallèlement au conduit (6; 16a, 16b) et s'étendant à travers le capot (3) vers l'extrémité frontale (1a), ledit conduit supplémentaire étant apte à délivrer de l'eau sur ladite lentille (2) pour la laver et la nettoyer de toute matière éventuellement déposée sur elle lors de l'utilisation du dispositif.

9. Le dispositif robotisé selon l'une quelconque des revendications précédentes, dans lequel ledit conduit flexible (6; 16a, 16b) a une flexibilité variable sur sa longueur, de façon que la partie dudit conduit émergeant du capot (3) à l'extérieur du corps capsulaire (1) comprend au moins deux portions ayant une flexibilité différente, une première portion adjacente audit capot (3) ayant une plus grande flexibilité au pliage, et une seconde portion comprise entre la première portion et l'extrémité dudit conduit ayant une plus faible flexibilité au pliage et une résistance mécanique supérieure.
